# EUROPEAN PATENT APPLICATION

(11) **EP 0 733 373 A2**
(43) Date of publication of application: **25.09.1996**
(21) Application number: 96302009.4
(22) Date of filing: 22.03.1996
(51) Int. Cl.: A61K 48/00, A61K 35/12, C12N 5/10

(54) **Compositions and methods for increasing the immunogenicity of tumor cells by administration of B7 and CD2-transfected cells**

(30) Priority: 23.03.1995 US 409768
(71) Applicant: Bristol-Myers Squibb Company, New York, N.Y. 10154 (US)
(72) Inventor: Chen, Lieping, Seattle, WA 98115-4928 (US); Hellström, Karl Erik, Seattle, WA 98105-5407 (US)
(74) Representative: Jones, Alan John

(57) **Abstract**

The present invention is directed to a method of inhibiting tumor cell growth. Tumor cells from a patient are transfected to express both B7 and a CD2 ligand on the surface of the transfected tumor cells and these cells are then readministered to the patient. The presence of the B7 and CD2 ligand molecules on the tumor cell surface stimulates a broad immunologic response against both the transfected and non-transfected tumor cells and results in the immunologic killing of localized and metastatic tumor cells. B7 and CD2 ligand transfected tumor cells, or cell membranes, serve as an immune enhancer to engender a potent immunologic response against the surface antigens present on the tumors cells.

SEQUENCE LISTING

(1) GENERAL INFORMATION:

(i) APPLICANT: CHEN, LIEPING
HELLSTROM, KARL ERIK

(ii) TITLE OF INVENTION: COMPOSITIONS AND METHODS FOR INCREASING
THE IMMUNOGENICITY OF TUMOR CELLS

(iii) NUMBER OF SEQUENCES: 2

(iv) CORRESPONDENCE ADDRESS:
(A) ADDRESSEE: BRISTOL-MYERS SQUIBB COMPANY
(B) STREET: 3005 FIRST AVENUE
(C) CITY: SEATTLE
(D) STATE: WASHINGTON
(E) COUNTRY: USA
(F) ZIP: 98121

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: POOR, BRIAN W.
(B) REGISTRATION NUMBER: 32,928
(C) REFERENCE/DOCKET NUMBER: ON0132

(ix) TELECOMMUNICATION INFORMATION:
(A) TELEPHONE: 206 727 3670
(B) TELEFAX: 206 727 3601

(2) INFORMATION FOR SEQ ID NO:1:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

GCTAAAGCTT CTCGAACCGC CACCATGTGC TTCATAAAAC AGGGAT 46

(2) INFORMATION FOR SEQ ID NO:2:

(i) SEQUENCE CHARACTERISTICS:
(A) LENGTH: 43 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

CGTAAAGCTT CTCGGAGTCT AGAGTTCCTT GTCAGGTTAA CAG 43

## Description

### Background of the Invention

A long-standing goal of cancer research has been to stimulate the immunological rejection of tumors. This goal is based on the hypothesis that tumors express foreign antigens which can potentially serve as targets for the immune system (Himmelweit, 1957, *The Collected Papers of Paul Ehrlich,* Pergamon Press, Oxford, England). Although it remains controversial to what extent spontaneous tumors express antigens which can be recognized as foreign by the immune system in conventional immunization and challenge experiments (Hewitt et al., 1976, *Br. J. Cancer **33**,* 241-259), it is well documented that many experimental tumors express antigens which can mediate tumor rejection in such experiments (Hellström and Hellström, 1991, "Principles of Tumor Immunity: Tumor Antigens," *In: The Biologic Therapy of Cancer*. DeVita, Jr. et al., Eds., J.B. Lippincott Co., Philadelphia, pp. 35-52, Boon, 1992, *Adv. Cancer Res. **58***:177-211).

Tumor immunity is primarily cell-mediated and circulating antibodies tend to play a minor role (Hellstrom, K.E. and Hellstrom, I., 1969, *Adv. Cancer Res.* ***12***:167-223). In some cases, the effector cells are CD8⁺ cytolytic T lymphocytes (CTL); while in other cases, CD4⁺ helper T (T_{H}) cells are the major effectors and work by amplifying CTL responses and acting in consort with macrophages and other cells (Melief, 1992, *Adv. Cancer Res*. ***58***:143-175; Greenberg, 1991, *Adv. Immunol*. ***49***:281-355). For a tumor antigen to be recognized by CTL, it has to be intracellularly processed into small peptides and presented via major histocompatability complex (MHC) class I molecules at the cell surface. To be recognized by CD4⁺ T cells, an antigen must be released from a tumor cell (since few tumors have MHC class II molecules), taken up and processed into peptides by antigen-presenting cells (APCs), then displayed on MHC class II molecules. Peptides generated from normal cellular proteins will be tolerated as "self," but peptides generated from viral proteins or mutated cellular proteins can be recognized as foreign and, therefore, trigger a T-cell response (Yewdell and Bennick, 1992, *Adv. Immumol. **52***:1-123).

Tumor immunology was largely ignored in the 1980s because most spontaneously occurring animal neoplasms failed to express any rejection antigens when tested by the conventional approach of immunization and challenge. However, the field is attracting much attention today. Recent findings partially responsible for the resurgence include (1) most cancers express mutated cellular oncogenes and/or mutated suppressor genes (Bishop, 1991, *Cell **64***:235-248; Levine et al., 1991, *Nature **351**,* 453-456); (2) some viruses, including human papilloma virus (HPV) type 16 and 18, play a role in the etiology of certain human tumors (Zur Hausen, 1991, *Science **254***:1167-1173) and can encode tumor specific rejection antigens (Chen et al., 1991, *Proc. Nat'l. Acad. Sci. USA **88***:110-114); (3) MHC class I molecules can present intracellular proteins as peptides (Yewdell and Bennick, 1992, *Adv. Immunol. **52:1***-123), which implies that proteins encoded by the mutated cellular oncogenes, suppressor genes and/or viral genes may be detectable by the immune system as non-self;" (4) genes encoding certain intracellular protein may be "silent" in most normal cells but activated in cancer cells where their product may be presented by MHC class I molecules and recognized as nonself (Van der Bruggen et al., 1991, *Science **254***:1643-1647).

These findings suggested that most spontaneous tumors appear to be non-immunogenic and may, therefore, reflect a lack of knowledge as to how to immunize against them rather than an inherent lack of molecules that can serve as targets for tumor destruction. Many factors contribute to the escape of tumors from immunologic control (Hellstrom, K.E. and Hellstrom, I., 1992, in *The Biologic Therapy of Cancer,* DeVita et al, eds., pp 35-52, J.B. Lippincott Co.). They include loss ofthe MHC and transporter molecules needed for antigen presentation by tumor cells; production by tumor cells of various immunosuppressive molecules, including transforming growth factor β (TGF-β); and the possible release of a tumor antigen which downregulates anti-tumor immunity, perhaps by activating suppressor cells. However, an additional explanation was needed as to why even highly antigenic tumors can grow in immunocompetent syngeneic hosts and are not immediately eliminated by an immune response. Recently it has been found that stimulation of a T cell response requires, in addition to an antigen-specific signal delivered through the T cell antigen receptor, a second antigen nonspecific or costimulatory signal delivered by accessory receptors following engagement by ligands expressed on antigen presenting cells. In the absence of such signaling, antigen-MHC complex interaction might lead to T cell clonal anergy or deletion (Mueller et al., 1989, *Annu. Rev. Immunol. **7***:455-480). Several molecules capable of providing costimulatory functions include the intracellular adhesion molecules (ICAMs), the lymphocyte function associated antigen (LFA)-3, the vascular cell adhesion molecule (VCAM)-1 and the head-stable antigen (HSA) (Liu and Linsley, 1993, *Curr. Opin. Immunol. **4***:265-270).

The B7 molecule, which interacts with its receptors CD28 and CTLA4 on T cells to provide a key signal for the generation ofT cell immunity, is expressed primarily on hematopoietic cells (Freeman et al., 1989, *J*. *Immunol. **143***:2714) and it is present only at very low levels, if at all, on many cultured human tumor cell lines. These findings suggest that one of the reasons why immunogenic tumors can often escape T cell destruction is that they lack appropriate costimulatory molecules. A prediction from this hypothesis is that introduction of costimulatory molecules into tumors which possess tumor rejection antigens would enhance their ability to induce specific anti-tumor immunity leading to tumor eradication in immunocompetent hosts.

The co-stimulatory molecule B7 has been used to transfect tumor cells (EP A-0 600 591; copending U.S. Application Serial No. not yet assigned, filed March 10, 1995, which is a file wrapper continuation of 08/161,183, filed December 1, 1993, which is a file wrapper continuation of 08/006,102, filed January 15, 1993, which is a continuation-in-part of 07/956,123, filed October 2, 1992 is incorporated by reference in its entirety; Chen et al., 1992, *Cell **71***:1093-1102; Townsend and Allison, 1993, *Science **259**;* 368-370; Baskar et al., 1993, *Proc. Nat'l. Acad. Sci. USA **90***:5687-5690). B7 transfected murine tumor cells which express a viral tumor rejection antigen (HPV 16 E7) were shown to have increased immunogenicity when injected into an immunocompetent syngeneic host. Surprisingly, following immunization with the B7⁺ E7⁺ transfected tumor cells, B7⁻E7⁺ tumor cells were also rejected. Weakly immunogenic tumor cells transfected with B7 have also been shown to have increased immunogenicity than that seen with the parental tumor cells. The rejection of non-transfected tumor cells following immunization with a B7 transfected tumor was also seen with these types of tumor cells. Interaction ofB7 with CD28 also has been found to costimulate cell-mediated cytotoxicity and proliferation of T cells initiated by either anti-CD2 or anti-CD3 monoclonal antibody (Azuma et al., 1992, *J. Erp. Med. **175***:353-360).

CD2 is a co-receptor involved in both T cell adhesion and activation (Moingeon et al., 1989, *Immunol. Rev. **111***:111 and Bierer and Burakoff, 1989, *Immunol. Rev. **111***:267). In the mouse, the natural ligand for CD2 has been defined as a 45 kDa glycoprotein designated CD48 (Kato et al., 1992, *J. Exp. Med. **176***:1241-1249). Interaction between murine CD2 and its ligand, CD48, are believed to be responsible for CD2-dependent adhesion in the murine system and also for T cell activation. In the human system, three CD2 ligands have been identified: CD58(LFA-3), CD59 and CD48 (Hahn et al., 1993, *in* Lymphocyte Adhesion Molecules, Shimizu ed., pp 105-134, R.G. Landes Co.). As in the murine system, human CD2 interaction with its ligand plays an important role in modulating and amplifying antigen-specific T cell responses.

Certain types of tumor cells still are considered non-immunogenic by standard immunologic assays. Transfection of these tumors with B7 alone does not appear to increase the immunogenicity of these tumor cells. In addition to lacking immunogenicity, these cells cannot efficiently induce tumor-specific CD8⁺ CTL in its syngeneic host. What is needed in the art are compositions and methods for increasing the immunogenicity of tumor cells, particularly weakly immunogenic or non-immunogenic tumor cells, and their ability to induce tumor specific cytotoxic T lymphocytes. The present invention provides such compositions and methods.

### Summary of the Invention

The present invention comprises compositions and methods for increasing the immunogenicity of tumor cells. In particular, the immune response to weakly or what are considered non-immunogenic tumors can be increased using the compositions and methods of the present invention. The compositions comprise tumor cells transfected with a gene encoding B7 and a gene encoding a CD2 ligand so as to express B7 and the ligand CD2 on the surface of the transfected tumor cells. Using these compositions *in vitro* and *in vivo* the immune response to both the transfected tumor cells and non-transfected tumor cells is increased. The tumor cells used for the transfections can be isolated from a tumor bearing patient.

Pharmaceutical compositions for use in increasing the immune response or immunogenicity of the tumor cells can comprise the transfected B7⁺CD² ligand⁺ tumor cells with a physiologically acceptable carrier. The transfected tumor cells can be alive or can be provided in an attenuated form. By attenuated is meant the cells are weakened so as to grow and proliferate much more slowly than the non-attenuated cells or they are dead. The compositions can also comprise membranes isolated from the B7⁺CD2 ligand⁺ transfected tumor cells combined with a physiologically acceptable carrier.

Methods for increasing the immune response or immunogenicity to a tumor cell are also encompassed by the present invention. In such methods, the tumor cells, particularly non-immunogenic or weakly immunogenic tumor cells, are transfected with a gene encoding B7 and a gene encoding a CD2 ligand such that B7 and the CD2 ligand are expressed on the surface of the transfected tumor cells. The B7⁺CD2 ligand⁺ transfected tumor cells are grown up and collected prior to administration to a tumor bearing patient in an immunogenically effective amount. The tumor cells in particular are non-immunogenic or weakly immunogenic and, even more particularly, are isolated from the tumor bearing patient. Methods of the present invention can also comprise administering attenuated transfected B7⁺CD2 ligand⁺ tumor cells or membranes isolated from the transfected tumor cells.

Further, the present invention comprises methods for stimulating a tumor-reactive cytotoxic T lymphocyte response. In these methods, tumor cells, particularly weakly immunogenic or non-immunogenic tumor cells, are transfected with a gene encoding B7 and a gene encoding a CD2 ligand such that B7 and the CD2 ligand are expressed on the surface of the transfected tumor cells. These transfected cells, either alive, in an attenuated form, or as cell membranes, are then administered to a tumor bearing patient in an immunogenically effective amount. Once an immune response to the tumor cells has been detected, lymphocytes can be isolated from the tumor bearing patient. The isolated lymphocytes are grown *in vitro* in the presence of tumor cells, either B7⁺CD2 ligand⁺ or parental tumor cells, and interleukin-2 to enhance a tumor-reactive cytotoxic T lymphocyte response. After enough CTLs have been obtained, the CTLs are formulated with a physiologically acceptable carrier and administered back to the tumor bearing patient. Various cytokines and interleukins can be combined with the CTLs to further enhance the cytotoxic response.

### Brief Description of the Drawings

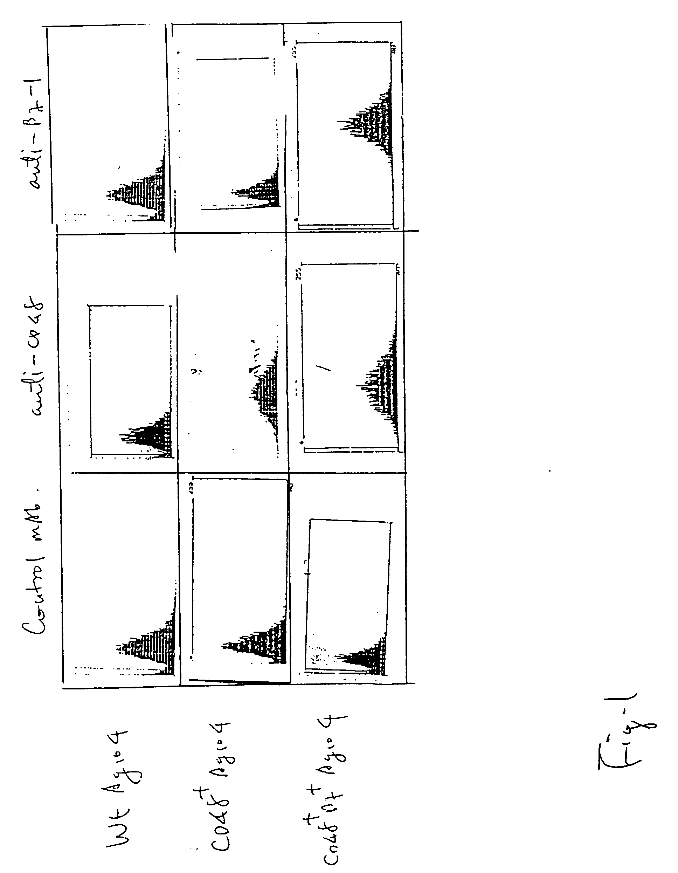

FIGURE 1 shows expression of CD48 and B7-1 molecules on Ag104 transfectants. Cells were stained with FITC-conjugated anti-CD48 mAb, anti-B7-1 mAb or the control mAb and were subjected to FACS analysis. A total of 5,000 cells were analyzed for each sample.

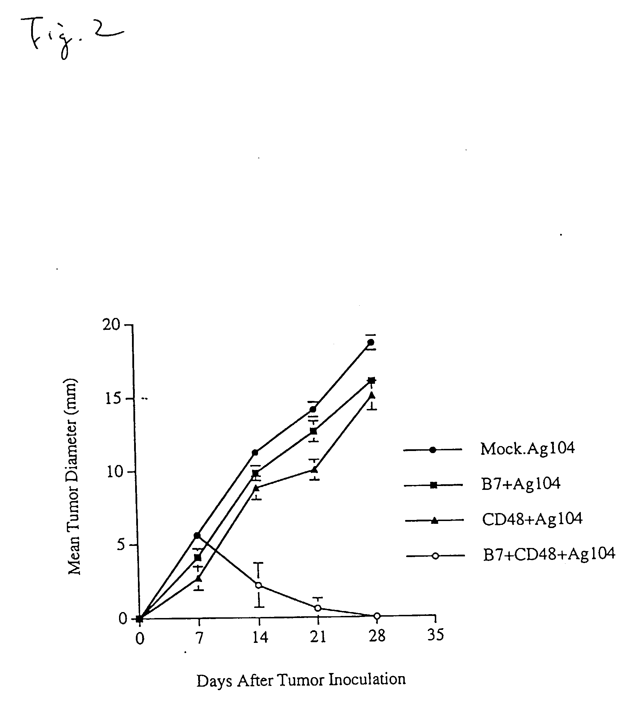

FIGURE 2 shows growth of tumors induced by CD48⁺ Ag104 cells in syngeneic mice. Mice were injected subcutaneously with the indicated cells at 1 x 10⁶ cells/mouse. Tumor size was assessed weekly by measuring perpendicular diameters with a caliper. The experiments were terminated when the tumors reached 20-30 mm in diameter, severe ulceration and bleeding had developed or the mice had died. The results are expressed as mean diameters (in mm) of tumors from groups of five mice each. Error bars represent the standard deviations of the means. Similar results were obtained in at least two experiments with each cell line.

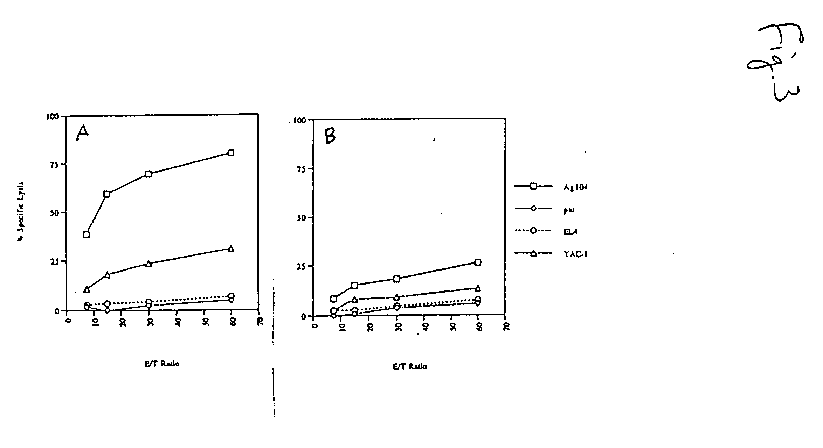

FIGURES 3A and 3B illustrate comparison of CTL activity generated from mice immunized with either CD48⁺ or CD48⁺B7⁺Ag104 cells and their specificity. Groups of five mice were injected with either CD48⁺Ag104 cells (B) or CD48⁺B7⁺Ag104 cells (A) at 1 x 10⁶ cells/mouse subcutaneously. Ten days later, tumor nodules were removed surgically. Spleen cells were prepared 14 days after injection, and were co-cultivated withy-irradiated wild-type Ag104 cells for 5 days. CTL activity against ⁵¹Cr-labeled target cells as indicated was measured.

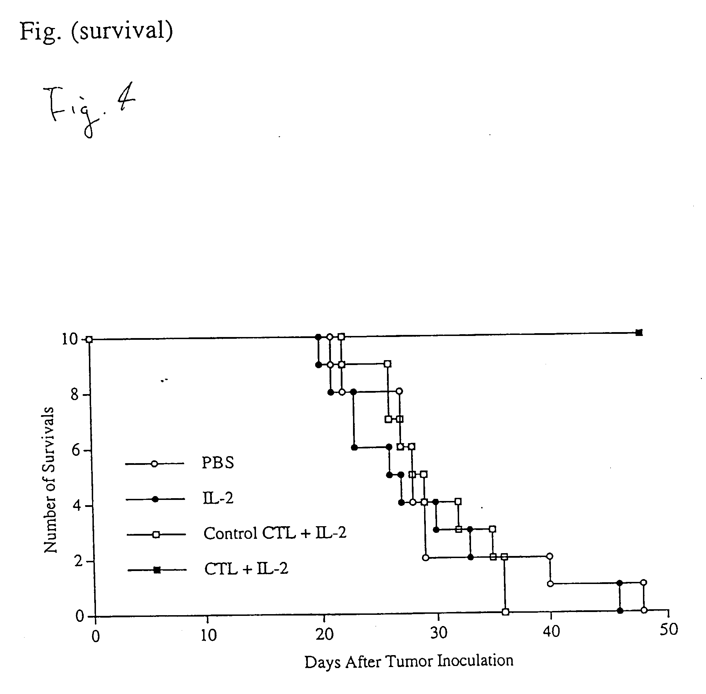

FIGURE 4 illustrates cure of three-day pulmonary micrometastasis of wild-type Ag104 cells by adoptive transfer of cytotoxic T cell clone CTL. The mice, in groups of 10, were injected intravenously with wild-type Ag104 cells at 1 x 10⁶ cells/mouse. Three days later, the mice were treated with 5 x 10⁶ cells of cytotoxic T cell line AlCTL by intravenous injection and followed the next day by injection intraperatoneally with 10⁴ U of recombinant human IL-2 once a day for 3 days. The control groups included mice injected with either saline alone, IL-2 alone or combination of a C3H-derived CTL line specific for H-2d antigens and IL-2. The mice were monitored daily for survival.

### Description of the Preferred Embodiments

The present invention is directed to compositions and methods for increasing the immunogenicity of tumor cells. In particular, the compositions and methods of the invention find use in increasing the immunogenicity of weakly immunogenic or non-immunogenic tumor cells so that a tumor bearing patient can have increased immune response to a tumor. Compositions of the present invention comprise tumor cells transfected with a gene encoding B7, the ligand for the costimulatory molecules CD28 and CTLA4 and a gene encoding a ligand for CD2 such that B7 and the CD2 ligand are expressed on the surface of the transfected tumor cells. Administration of the transfected (B7⁺CD2 ligand⁺) tumor cells into a tumor bearing patient increases the immunogenicity of the tumor cells stimulating the immune system to mount a response to both transfected and, most importantly, non-transfected tumor cells.

Tumor cells can be melanoma, carcinoma, including for example, carcinoma of the breast, colon, pancreas, bladder, ovary, etc., or sarcoma among others. The source of the tumor cells can be from a tumor bearing patient or from a tumor cell bank, such as the American Type Culture Collection. Immunogenicity of the tumor cells is determinable by methods well know to the skilled artisan, but basically requires immunizing a host with a certain number of tumor cells and determining the extent of the immune response by the host to the tumor cells. The assay can be by, for example, a standard ⁵¹Cr cytolytic assay (Chen et al., 1992, *J. Immunol. **148***:2617-2621). A non-immunogenic or weakly immunogenic tumor is one wherein either no immune response can be detected or a very low response is detected.

B7, which can include either B7-1 (CD80) and B7-2 (B70, CD82), is known to be a co-stimulator in the induction of T cell-mediated antitumor immunity (Chen et al., 1993, *Immunol. Today **14***:483-486; G. Yang et al., 1995, *J. Immunol. **154**:*2794-2800). The construction of vectors encoding B7 and their use in transfecting tumor cells has been described. It is also known that non-immunogenic tumors and weakly immunogenic tumor cells transfected with B7 were unable to induce either protective immunity or an increased tumor-reactive cytolytic T cell response.

In the present invention, a DNA fragment encoding the entire open reading frame of a CD2 ligand can be amplified by reverse transcription-coupled polymerase chain reaction (PCR) as described by Chen et al., 1991, *Proc. Natl'l. Acad. Sci. USA **88***:110-114. Sense and antisense primers are designed to include restriction sites for manipulation of the various DNA fragments. As a model for the human system a DNA fragment encoding the murine CD2 ligand was used. In the mouse, CD48 has recently been associated with the T cell receptor CD2 (Kato et al., 1992, *J. Exp. Med. **176***;1241-1249). Binding of anti-CD2 monoclonal antibody to the T cell receptor CD2 has long been known to activate T cells and to induce their proliferation (Bierer and Hahn, 1993, *Seminar in Immunol. **5***:249-261).

In the case of the human, a DNA sequence encoding a human CD2 ligand, such as LFA-3(CD58), CD59 or CD48 would be used. Once the described DNA sequence encoding the CD2 ligand has been amplified, the fragment can be inserted into one of many vectors available in the art including pRC/CMV, pLXSHD or CDM8. The vector can include the coding region for B7 or for the CD2 ligand, or could encode both depending on the amount of heterologous DNA the particular vector chosen is able to accommodate. Methods for the genetic manipulations are well known to the skilled artisan *(See,* Sambrook et al, 1989, *Molecular Cloning, A Laboratory Manual,* 2nd Ed., Cold Spring Harbor Laboratories, incorporated herein by reference in its entirety). The expression vector can then be transferred into the tumor cell by electroporation, CaCl₂ precipitation, injection or by other well known techniques.

The B7 and CD2 ligand construct can also be introduced into the tumor cells by other methods including using retroviruses (Kuriyama et al., 1991, *Cell Struc. and Func. **16***:503-510), or immunoliposomes (Martin and Papahadipoulos, 1982, *J. Biol. Chem. **257***:286-288) as vehicles for introduction of the vector. One of skill in the art will recognize that many other methods are available and that the present invention is not limited by the specific method used.

Once a population of tumor cells has been transfected with the vectors encoding B7 and CD2 ligand, it is then necessary to screen for those transfected tumor cells which express on their surface both B7 and the CD2 ligand. Screening for transfected cells can be carried out by such methods as flow- cytometry analysis, where antibodies specific for B7 and for the CD2 ligand are labeled with a fluorescent molecule and those cells which are positive for both B7 and CD2 ligand are separated from the non-transfected and singly transfected tumor cells. Successfully transformed cells can also be identified by other well known methods including analyzing the DNA content of the transfected cells, an ELISA assay or any other antibody based assay system. Other assays capable of testing for B7 and CD2 ligand expression on the surface of the transfected tumor cells would be known to be skilled artisan.

B7⁺ and CD2 ligand⁺ transfected tumor cells of the present invention can be used directly in the formulation of a pharmaceutical composition which can be administered back to the tumor bearing patient to increase the patient's immune response to a tumor. In such a formulation, the transfected tumor cells can be combined with a physiologically acceptable carrier. By a physiologically acceptable carrier is meant an aqueous solution which approximates physiological conditions. A variety of physiologically acceptable carriers are known in the art and can be used, *e.g.,* water, buffered water, various concentrations of saline, glycine, and the like. Auxiliary substances can also be added, as required, to approximate physiological conditions such as pH adjusting and buffering agents, toxicity adjusting agents, and the like, for example, sodium acetate, sodium chloride, potassium chloride, calcium chloride, sodium lactate, etc.

The transfected tumor cells can also be used in an attenuated form. By attenuated is meant that the transfected tumor cells have been weakened or killed so as to reduce or stop their ability to proliferate. Methods for accomplishing this weakened or killed state are well known in the art and can include treating the transfected cells with chemical agents, heat or radiation. For example, a sample of transfected tumor cells can be γ-irradiated with about 3,000 to 15,000 rad of γ radiation for 2 to 10 minutes. This treatment kills the cells without damaging or altering the immunogenic or antigenic epitopes of the molecules on the surface of the transfected tumor cells.

Cellular membranes from the transfected tumor cells ofthe present invention can also be used in the formulation of pharmaceutical compositions. The transfected tumor cells can be disrupted or lysed by any one of several methods known in the art, such as sonication, a French press, or other mechanical means, or by using enzymes or other chemicals, etc. Once the transfected tumor cells have been disrupted, the cellular membranes can be isolated such as by gradient centrifugation, differential centrifugation or the like. The collected membranes can then be formulated with a physiologically acceptable carrier to provide a composition which can be administered back to a tumor bearing patient.

In treating a patient, the compositions of the present invention would most likely be used as an adjuvant to existing cancer treatments, such as surgery, radiation therapy and chemotherapy. Once the majority of the tumor load has been removed, the compositions of the present invention can be administered in an immunogenically effective amount to enhance the immune response of the tumor bearing patient. This enhanced immune response would to slow or stop the recurrence of a tumor, the growth of metastatic lesions or regrowth of tumor cells which were not destroyed by the initial therapy. This proposed use of the present invention is not intended to limit the use of the compositions of the present invention as a secondary or adjunctive therapy, but is intended to provide a framework for how the compositions would be used initially.

The precise amount or number of transfected tumor cells or transfected tumor cell membranes constituting an immunogenically effective amount necessary to enhance the immune response will depend on the patient's state of health, the immunogenicity of the transfected tumor cell and other factors. Single or multiple administrations can be carried out with dosage levels and patterns of administration being selected by the treating physician. Methods are well known in the art on how to monitor a patient's immune response to the compositions of the present invention and protocols as to how to adjust dosage levels are also well known. In any event, the pharmaceutical composition should provide a quantity of B7⁺ and CD2 ligand⁺ transfected tumor cells or tumor cell membrane sufficient to effectively elevate the patient's immune response to a tumor.

In a preferred method for treating a tumor bearing patient, a tumor can be removed from the patient as part of the primary treatment regime for the cancer. The tumor can then be broken up into individual cells. A single cell suspension of tumor cells can then be transfected with a gene encoding B7 and a gene encoding CD2 ligand such that B7 and CD2 ligand are expressed on the surface of the transfected tumor cells. Cells expressing both B7⁺ and CD2 ligand⁺ on their surface are selected and formulated with a physiologically acceptable carrier prior to administration back to the tumor bearing patient. Depending on the patient's condition, the treating physician would then determine the dosage required for an immunogenically effective amount of transfected B7⁺ and CD2 ligand⁺ tumor cells. The compositions can be administered in either a single administration or in multiple administrations at the physician's discretion. The patient would be monitored by standard methods to determine the elevation of immune response to the tumor which has been obtained.

Another therapeutic method of the present invention is a method for stimulating a tumor-reactive cytotoxic T lymphocyte response. The method comprises transfecting a tumor cell, preferably removed from a tumor bearing patient, with a gene encoding B7 and a gene encoding a CD2 ligand such that B7 and CD2 ligand are expressed on the surface of the transfected tumor cell. This transfected tumor cell expressing B7 and CD2 ligand on its surface would be administered back to the tumor bearing patient in an immunogenically effective amount. After a period of time, the patient would be monitored for an immune response to the tumor cells and after a sufficient response was detected, lymphocytes would be isolated from the tumor bearing patient. The isolated lymphocytes can then be grown *in vitro,* preferably in the present of nontransfected or transfected tumor cells and a lymphokine or cytokine, *e.g.,* interleukin-2, to increase the number of tumor-reactive cytotoxic T lymphocytes. The activated lymphocytes are collected and combined with a physiologically acceptable carrier. A therapeutically effective dose of tumor-reactive cytotoxic T lymphocytes is then administered back to the tumor bearing patient. By therapeutically effective dose is meant a sufficient number of cytotoxic T lymphocytes to enhance tumor cell killing *in vivo.* Adoptive immunotherapy methods such as those described above are know in the art *(See,* for example, Rosenberg et al., 1986, *Science **233***:1318-1321). Therefore, alterations and modifications of the above-described method are anticipated and would be recognized by the skilled artisan.

The following experimental data and information are offered by way of example and are not intended nor are they to be construed as limitations to the scope of the present invention.

### Examples

### Mice and Cell Lines.

Female C3H/HeN (C3H) mice, 4-6-weeks old, were purchased from the Jackson Laboratory (Bar Harbor, ME). Ag104, a spontaneous fibrosarcoma of C3H (H-2^{k}) origin (Ward et al., 1989, *J. Exp. Melt **170***:217-232), was provided by Dr. Hans Schreiber of University of Chicago. The EL4 thymoma (ATCC TIB 40) is of C57BL/6 (H-2^{b}) origin. The R1.1 lymphoma (ATCC TIB 42) is derived from DBA2 (H-2^{d}) mice. YAC-1 (ATCC TIB 160) is a natural killer-sensitive lymphoma of ASn (H-2^{a}) origin. ATCC designated tumor lines were obtained from American Type Culture Collection, Rockville, Maryland. Once acquired, all tumors were briefly expanded *in vitro* and frozen to decrease experimental variation. All cells were maintained *in vitro* at 37°C in DMEM containing 10% fetal calf serum (FCS, HyClone, Logan, Utah) for no longer than one month before *in vivo* experiments.

### Cloning and Transfection of Murine CD48

A DNA fragment encoding the entire open reading frame of murine CD48 was amplified by reverse transcription-coupled PCR (Chen et al., 1991, *Proc. Nat'l. Acad*. *Sci. USA* **88**:110-114) from total RNA prepared from EL4 cells. The sense primer 
(5'- GCTAAAGCTTCTCGAACCGCCACCATGTG CTTCATAAAACAGGGAT-3');
(SEQ ID NO:1)
 consists of an oligonucleotide corresponding to 51-72 nucleotides of murine CD48 cDNA (Wong et al., 1990, *J. Exp. Med. **171***:2115-2130) plus restriction sites for Hind III and XhoI. The antisense primer 
(5'- CGTAAAGCTTCTCGGAGTCTAGAGTTCCTTGTCA GGTTAACAG-3');
(SEQ ID NO:2)
 corresponds to 762-773 nucleotides of murine CD48 cDNA plus sites for Hind III, XhoI, and XbaI. The PCR product was directly cloned into the vector pRC/CMV (Invitrogen, San Diego, CA). The sequence of murine CD48 was verified by DNA sequencing analysis. Ag104 cells were transfected with this vector by electroporation and the transfectants were selected in medium containing 1 mg/ml G418 (Life Technologies, Grand Island, NY). The B7⁺ Ag104 cells were also transfected with the vector and positive clones were sorted by FACS as described (Chen et al., 1992, *Proc. Nat'l. Acad. Sci. USA **88***:110-114).

### Antibodies

Purified and fluorescein isothiocyanate- (FITC-) or PE-conjugated (phycoerytherin-) monoclonal antibodies 1G10 (anti-mouse B7-1, PharminGen, San Diego, CA), OX-78 (anti-mouse CD48, rat IgG2a, BioSource International, Camarillo, CA). MAb 10.2 (anti-human CD5) was used as a control.

### Immunostaining and Flow Cytometry Analysis

Single cell suspensions were incubated at 4°C for 30 min with FITC-conjugated mAb and/or PE-conjugated mAb at 10 µg/ml, washed twice with DMEM containing 10% FCS, and analyzed on a Couler Epics C IV cytometer.

### Generation and assay of Cytotoxic T Lymphocyte activity.

The methods for inducing and assaying for a CTL response to tumor cells have been described (Chen et al., 1992, *J. Immunol. **148***:2617-2621) and were used with minor modification. Briefly, C3H mice in 4 groups (n=5) were injected subcutaneously with 1 x 10⁶ cells/mouse of mock transfected, or B7⁺, CD48⁺, or B7⁺CD48⁺ transfected Ag104 cells, respectively. Ten days later, growing tumor nodules were excised. After 7-21 days, mice from each group were sacrificed and a single cell suspension was prepared from the spleen. The spleen cells were then cocultivated with γ-irradiated wild type Ag104 (non-transfected) cells (10,000 rads) in 24-well plate (Costa, Cambridge, MA) for 5 days. Long-term T cells lines were generated by restimulating the spleen cell cultures every 7-10 days with γ-irradiated B7⁺CD48⁺ Ag104 cells (10,000 rads) and γ-irradiated splenocytes (3,000 rad) from C3H mice in the presence of human recombinant IL-2 (Cetus, CA) at 5 U/ml as described (Chen et al., 1992, *J. Immunol. **148***:2617-2621). The cytolytic activity of lymphocytes was examined in a standard 4 hr ⁵¹Cr release assay at different E/T ratios as indicated in the figures. For antibody blocking experiments, mAbs were included during the 4 hr culture.

### Tumorigenicity studies

Tumorigenicity of transfectants was assayed by injecting 1 x 10⁶ cells into the right back of mice in a 0.1 ml volume via a 26-gauge needle with a 1 ml syringe. The mice were scored for tumor growth every 4-8 days and tumor size was documented by measuring two perpendicular diameters in mm using a caliper.

### Adoptive immunotherapy of established micrometastasis induced by Ag104 with tumor specific Cytotoxic T Lymphocytes.

These experiments followed a procedure as described (Kahn et al., 1991, *J. Immunol. **146***:235-341) with minor modifications. Briefly, C3H mice, in groups of 10, were administered 1 x 10⁵ Ag104 tumor cells/mouse intravenously. Three days later, the mice were injected intravenously with 5 x 10⁶ cells of a tumor specific CTL line (A1CTL) or of a C3H-derived anti-H-2^{d} CTL line (Grabstein, 1980, *in* Selected Methods in Cellular Immunology, Michell et al., eds. pp 124-127, W.H. Freeman & Co.) followed at day 4 by injection intraperitoneally of 10⁴ U of rIL-2 (Cetus Corp., Emeryville, CA) every day for three days. Other groups of mice were either treated with the same dosage of rIL-2 only, or left untreated. The mice were monitored for survival daily and when a mouse was found dying, the mouse was sacrificed and the lungs removed for confirmation of metastasis.

### Results and Discussion

Ag104 is a spontaneously-raised sarcoma from an aged C3H/HeN mouse and can not induce a protective immunity by a variety of immunization and challenge procedures (Chen et al., 1994, *J. Exp. Med. **179**:523-532).* Expression of B7-1 costimulator by gene transfer did not make Ag104 cells immunogenic and the immunization with Ag104 cells expressing B7-1 are not capable of eliciting a CTL response (Chen et al., 1994, *J. Exp. Med. **179***:523-532), demonstrating the poorly-immunogenic nature of this sarcoma line.

FACS analysis of the expression of MHC and costimulatory molecules by specific mAbs revealed that Ag104 line expresses high level of both H-2K^{k}, D^{k} and vascular cell adhesion molecule 1 (VCAM-1), but it does not express MHC class II and several potential costimulatory molecules, such as B7-1, B7-2, CD48, intercellular adhesion molecule 1, 2 (ICAM-1/II) and heat stable antigen (HSA) (data not shown).

A recombinant plasmid, pRC/CMV-mCD48, containing the murine CD48 gene were constructed. This vector used a CMV promoter to drive CD48 open reading frame expression and also contains the neomycin-resistance gene for drug selection. Tumor lines, including wild-type Ag104 or B7-1⁺ Ag104, (Chen et al., 1994, *J. Exp. Med.* 179:523-532) were transfected with this vector and the clones obtained from each line were screened by FACS for expression of CD48. Three to 5 clones from each tumor line which stably expressed CD48 and/or B7-1 on their surface were picked for further experiments. After *in vivo* tumorigenicity tests (see Fig. 2), one clone from each line was selected for further experiments. The CD48 and B7-1 expression in several representative clones were shown in Fig. 1.

As shown in Figure 2, the wild-type Ag104 tumor grew progressively in all mice inoculated. Tumor also grew progressively in mice inoculated with CD48⁺Ag104 and B7⁺Ag104 transfected tumor cells. In contrast, a transfectant expressing both CD48 and B7-1, namely B7⁺CD48⁺Ag104, completely regressed after a transient growth in syngeneic C3H/HeN mice. These data demonstrate that synergy between CD48 and B7-1 is required in the induction of a efficient host response for inhibition of Ag104 tumor growth.

To demonstrate that decreased tumorigenicity of CD48⁺B7⁺Ag104 tumor line is associated with the induction of specific CTL response, splenocytes from the mice immunized with the cotransfectants were harvested and further stimulated *in vitro* by co-cultivation with irradiated wild-type Ag104 cells and IL-2 for 5 days. Subsequently, they were tested for CTL activity in a standard ⁵¹Cr release assay. As indicated in Figure 3, bulk-cultured spleen cells from the mice immunized with CD48⁺B7⁺Ag104 cells revealed a high level of CTL activity against wild-type Ag104 cells (Figure 3A). In contrast, bulk cultured spleen cells from the mice immunized by CD48⁺Ag104 (Figure 3B) or B7⁺ Ag104 (data not shown) did not show any significant CTL activity to wild-type Ag104 targets.

The bulk-cultured CTL from B7⁺CD48⁺Ag104-immunized mice also showed a natural killer-like activity since they lysed a natural killer-sensitive YAC-1 cell line at a moderate level. However, Ag104 is resistant to natural killer cell-mediated lysis (data not shown), indicating that lysis of wild-type Ag104 is mediated by specific cytolytic T cells. This notion is further supported by resistance of several syngeneic and allogenic tumor lines, such as K1735-M2 melanoma and EL4 lymphoma, to lysis of bulk-cultured CTL (Figure 3A).

To determine whether the cytotoxic T lymphocytes induced from the mice immunized with co-transfected CD48⁺B7⁺Ag104 line can be propagated *in vitro* to induce a potent CTL line, we repeatedly stimulated the spleen cells from the mice immunized with CD48⁺B7⁺Ag104 line and tested CTL activity in a 10-month period. A representative CTL line, AlCTL, showed high specificity against Ag104 cells and did not lyse several other tumor lines, including YAC-1 cells. We have also found that of the AlCTL line, >99% cells are CD8⁺ (data not shown). These data demonstrate that a CTL line specific for Ag104 can be propagated *in vitro* for extended periods and cytolytic activity is enhanced.

To test whether the propagated cytotoxic T lymphocytes can be utilized for treatment of established wild-type tumors, we employed a pulmonary micrometastasis model. As shown in Figure 4, the injection of C3H/HeN mice intravenously with wild-type Ag104 cells at 1 x 10⁶ cells/mouse led to death of all the mice within approximately 50 days. However, the mice treated with AlCTL line and IL-2 were alive for over 60 days. In contrast, the mice treated with either IL-2 or IL-2 combined with a control CTL line did not prolong the survival of the mice (Figure 4). The mice which died were examined thoroughly for gross pathology and were found to have died of tumor growth in the lungs.

Co-expression of CD48 with B7-1 molecules on a poorly-immunogenic sarcoma Ag104 has made possible the induction of a potent CTL response to tumor antigen. Thus, even poorly-immunogenic tumor cells express tumor-specific antigens which can serve as a target for CTL *in vitro* and *in vivo* although these potential antigens did not induce efficient CTL response *in vivo.* Furthermore, the cytotoxic T lymphocytes obtained can be propagated *in vitro* and can further be used for adoptive immunotherapy to potentially cure a tumor or prevent regression of a tumor following initial treatment.

The foregoing description and examples are intended as illustrative of the present invention, but not as limiting. Numerous variations and modifications may be effected without departing from the true spirit and scope of the present invention.

## Claims

1. A composition comprising tumor cells transfected with a gene encoding B7 and a gene encoding a CD2 ligand so as to express B7 and the CD2 ligand on the surface of the transfected tumor cell.

2. The composition of claim 1, wherein the CD2 ligand is human LFA-3, CD58, CD59 or CD48.

3. The composition of claim 1, wherein the tumor cells are isolated from a patient tumor.

4. The composition of claim 1, wherein the transfected tumor cells are attenuated.

5. The composition of claim 1, wherein the tumor cells are non-immunogenic or weakly immunogenic.

6. A pharmaceutical composition for use in stimulating an immunogenic response to a tumor comprising tumor cells transfected with a gene encoding B7 and a gene encoding a CD2 ligand so as to express B7 and the CD2 ligand on the surface of the tumor cells and a physiologically acceptable carrier.

7. The pharmaceutical composition of claim 6, wherein the CD2 ligand is human LFA-3, CD58, CD59 or CD48.

8. The pharmaceutical composition of claim 6, wherein the tumor cells are isolated from a patient tumor.

9. The pharmaceutical composition of claim 6, wherein the transfected tumor cell is attenuated.

10. The pharmaceutical composition of claim 6 wherein the tumor cells are non-immunogenic or weakly immunogenic.

11. A composition comprising cell membranes isolated from a tumor cell transfected with a gene encoding B7 and a gene encoding a CD2 ligand so as to express B7 and the CD2 ligand on the surface of the transfected tumor cell.

12. The composition of claim 11, wherein the CD2 ligand is human LFA-3, CD58, CD59 or CD48.

13. A method for increasing the immunogenicity of a tumor cell comprising:
(a) transfecting the tumor cell with a gene encoding B7 and a gene encoding a CD2 ligand such that B7 and the CD2 ligand are expressed on the surface of the transfected tumor cell; and
(b) administering to a tumor-bearing patient an immunogenically effective amount of transfected B7⁺ CD2 ligand⁺ tumor cells.

14. The method of claim 13, wherein the CD2 ligand is human LFA-3, CD58, CD59 or CD48.

15. The method of claim 13, wherein the tumor cell is isolated from the tumor bearing patient.

16. The method of claim 13, wherein the transfected tumor cell is attenuated.

17. The method of claim 13, wherein the tumor cell is non-immunogenic or weakly immunogenic.

18. A method for stimulating a tumor-reactive cytotoxic T lymphocyte response comprising:
(a) transfecting tumor cells with a gene encoding B7 and a gene encoding a CD2 ligand such that B7 and the CD2 ligand are expressed on the surface of the transfected tumor cells;
(b) administering to a tumor-bearing patient an immunogenically effective amount of the transfected tumor cells;
(c) isolating lymphocytes from the tumor-bearing patient having an immunogenically effective response to the tumor cells;
(d) growing the isolated lymphocytes *in vitro* in the presence of tumor cells or transfected tumor cells and interleukin 2 to increase the number of tumor-reactive cytotoxic T lymphocytes; and
(e) administering a therapeutically effective dose of the tumor-reactive cytotoxic T lymphocytes to the tumor bearing patient.

19. The method of claim 18, wherein the CD2 ligand is human LFA-3, CD58, CD59 or CD48.

20. The method of claim 18, wherein the transfected tumor cells are attenuated.

21. The method of claim 18, wherein the tumor cells are non-immunogenic or weakly immunogenic.

22. A compound according to any one of Claims 1 to 5, or a pharmaceutical composition in accordance with any one of Claims 6 to 12, for use in the treatment of cancer.
